# EUROPÄISCHE PATENTANMELDUNG

(11) **EP 1 219 955 A1**
(43) Veröffentlichungstag der Anmeldung: **03.07.2002**
(21) Anmeldenummer: 01117158.4
(22) Anmeldetag: 14.07.2001
(51) Int. Cl.: G01N 21/898, G01N 33/46

(54) **Verfahren zur optischen Qualitätsdokumentation eines Holzkörpers**

(30) Priorität: 23.12.2000 DE 10064891
(71) Anmelder: Rettenmeier Holding AG, 91634 Wilburgstetten (DE)
(72) Erfinder: Funk, Michael, 73479 Ellwangen (DE)
(74) Vertreter: Lorenz, Werner, Dr.-Ing.

(57) **Zusammenfassung**

Bei einem Verfahren zur Dokumentation, insbesondere zur optischen Qualitätsdokumentation, eines Holzkörpers (1), insbesondere eines an ein Sägewerk gelieferten unbearbeiteten und/oder entrindeten Rundholzabschnittes ist vorgesehen, daß der Holzkörper (1) mittels wenigstens einer Bilderzeugungsvorrichtung (2a,2b) erfaßt und als Bilddokument (7) abgespeichert wird.

## Beschreibung

Die Erfindung betrifft ein Verfahren zur Dokumentation, insbesondere zur optischen Qualitätsdokumentation, eines Holzkörpers.

Zur Ermittlung des Wertes bzw. des Kaufpreises von Holzkörpern werden die in das Sägewerk bzw. die Sägeindustrie gelieferten Holzkörper über eine Werkseingangsvermessungsanlage optoelektronisch, röntgonologisch oder mittels Ultraschall vermessen. Somit kann jedem Holzkörper eine Länge, ein Durchmesser und daraus hergeleitet ein Volumen zugeordnet werden. Neben dem Volumen des Holzkörpers spielt dessen Qualität zur Ermittlung des Kaufpreises eine entscheidende Rolle. Die Qualität bzw. die Gütesortierung erfolgt dabei zum einen durch den Lieferanten im Wald oder zum anderen durch den Kunden bzw. das Sägewerk im holzverarbeitenden Betrieb. Die Bestimmung der Qualität wird im wesentlichen anhand von äußeren Kriterien bzw. dem äußeren Erscheinungsbild des Holzkörpers durchgeführt.

Nachteilig bei diesem Verfahren ist, daß sich zwar das Volumen des Holzkörpers exakt bestimmen und dokumentieren läßt, eine Dokumentation der Qualität des Holzes jedoch nicht möglich ist. Dies hat, insbesondere wenn das Holz im Werk sortiert und somit mit anderen Hölzern zusammen gelagert wird, zu Differenzen zwischen dem Lieferanten und dem Abnehmer der Holzkörper geführt, da sich Lieferant und Abnehmer im Streitfall nicht über die werkseitig festgestellte Qualität einigen konnten. Eine Klärung dieser Differenzen, insbesondere wenn die betreffenden Holzkörper bereits mit anderen Hölzern gelagert bzw. verarbeitet wurden, ist im nachhinein nicht möglich.

Der vorliegenden Erfindung liegt daher die Aufgabe zugrunde, ein Verfahren zu entwickeln, das die Nachteile des Standes der Technik löst, das einfach, schnell und kostengünstig durchführbar ist und eine nachträgliche Qualitätsprüfung der Holzkörper ermöglicht.

Diese Aufgabe wird erfindungsgemäß durch die Merkmale von Anspruch 1 gelöst.

Durch die Entwicklung des erfindungsgemäßen Dokumentationsverfahrens für Holzkörper, insbesondere für Rundholzabschnitte, die an Sägewerke geliefert werden und deren Qualität mittels wenigstens einer Bilderzeugungsvorrichtung erfaßt und als Bilddokument abgespeichert wird, läßt sich die Güte bzw. die Qualität in einfacher Weise zuverlässig und eindeutig dokumentieren. Nach der Erfassung des Holzkörpers durch die wenigstens eine Bilderzeugungsvorrichtung kann der Holzkörper in bekannter Weise im Sägewerk bzw. in der Sägeindustrie mit anderen Holzkörpern zusammen gelagert, respektive verarbeitet werden. Falls mit dem Lieferanten des Holzkörpers im nachhinein Uneinigkeit über die Qualität der Lieferung besteht, läßt sich diese in einfacher Weise anhand der abgespeicherten Bilddokumente nachvollziehen bzw. dokumentieren. Die Bestimmung des Wertes bzw. des Kaufpreises des Holzkörpers ist somit zuverlässig und nachvollziehbar gewährleistet.

Die Dokumentation läßt sich in einfacher Weise durch die Bilderzeugungsvorrichtungen schnell und kostengünstig erstellen. Ein Abspeichern der erfaßten Holzkörper in Form eines Bilddokumentes hat sich in Versuchen als schnell, einfach und kostengünstig durchführbar erwiesen. Darüber hinaus ist die Lagerung dieser Bilddokumente kostengünstig und ein Abruf der Bilddokumente jederzeit in gleichbleibender Qualität möglich.

Erfindungsgemäß kann ferner vorgesehen sein, daß eine vordere Stirnseite und/oder eine hintere Stirnseite des Holzkörpers von jeweils wenigstens einer stirnseitigen Bilderzeugungsvorrichtung erfaßt wird.

In der Praxis hat sich gezeigt, daß sich mit einem derartigen Aufnahmeverfahren eine besonders gute Dokumentation der Qualität des Holzkörpers erstellen und abspeichern läßt.

Von Vorteil ist es, wenn der Holzkörper von wenigstens jeweils einem, vorzugsweise drei, längsseitigen Bilderzeugungsvorrichtungen wenigstens an einer Längsseite und/oder der Oberseite des Holzkörpers erfaßt wird.

Ein derartiges Verfahren zum Erfassen der Qualität des Holzkörpers hat sich als besonders geeignet bezüglich einer späteren Dokumentation herausgestellt. Ein Erfassen des Holzkörpers an einer Längsseite und in einem Winkel von 90° auf die Oberseite mittels jeweils drei Bilderzeugungsvorrichtungen hat sich als besonders zweckmäßig und kostengünstig erwiesen. Eine Erfassung von mehreren Längsseiten ist selbstverständlich problemlos möglich, jedoch unter Berücksichtigung der Kosten und des erhöhten Speicherbedarfs nicht anzustreben, insbesondere weil dadurch der Aussagegehalt der Dokumentation nicht wesentlich erhöht wird.

In einer Ausgestaltung der Erfindung kann vorgesehen sein, daß beim Passieren des Holzkörpers durch eine Lichtschranke ein Ausgangssignal zum Erfassen bzw. Aufnehmen des Holzkörpers durch wenigstens einen Teil der Bilderzeugungsvorrichtung ausgelöst wird.

Durch eine Lichtschranke läßt sich das Auslösen der Bilderzeugungsvorrichtungen gezielt steuern, so daß die unterschiedlichen Holzkörper jeweils mit derselben Qualität erfaßt werden können. Die Bilderzeugungsvorrichtungen können dabei in vorteilhafter Weise auf die bekannten Abstände zu dem Holzkörper eingestellt werden. Dabei kann auch vorgesehen sein, daß die Lichtschranke alle oder lediglich einen Teil der Bilderzeugungsvorrichtungen auslöst. Die weiteren noch vorhandenen Bilderzeugungsvorrichtungen können bei Bedarf entweder durch eine weitere Lichtschranke oder durch eine gezielte Verzögerung ausgelöst werden.

In einer weiteren Ausgestaltung der Erfindung ist es auch möglich, daß die stirnseitigen Bilderzeugungsvorrichtungen unabhängig voneinander ausgelöst werden, so daß beispielsweise lediglich die hintere Stirnseite des Holzkörpers oder die vordere Stirnseite des Holzkörpers erfaßt wird.

Von Vorteil ist es, wenn das Bilddokument digital, vorzugsweise im JPEG-Format, gespeichert und mit einem Identifizierungsschlüssel versehen wird.

Eine digitale Speicherung des Bilddokumentes hat sich bezüglich des Speicheraufwands und einer einfachen und kostengünstigen Speicherung als besonders geeignet herausgestellt. Eine Abspeicherung im JPEG-Format ermöglicht dabei ein einfaches Betrachten des Bilddokumentes mittels herkömmlicher Programme. Darüber hinaus läßt sich ein im JPEG-Format gespeichertes Bilddokument in einfacher Weise per Datenträger oder via Internet dem Lieferanten zur Verfügung stellen.

Der Identifizierungsschlüssel bzw. der Dateiname kann dabei beispielsweise durch eine Fuhrenkennung und/oder die verwendeten Bilderzeugungsvorrichtungen und/oder eine fortlaufende Numerierung gebildet werden. Somit läßt sich das Bilddokument einer entsprechenden Fuhre eines Lieferanten zuordnen. Bei auftretenden Differenzen bezüglich der Qualität der gelieferten Holzkörper können die zugehörigen abgespeicherten Bilddokumente in einfacher Weise aufgefunden und zur Dokumentation der Qualität der Fuhre bzw. der Qualität der einzelnen Holzkörper verwendet werden. Darüber hinaus gibt der Identifizierungsschlüssel Aufschluß darüber, welche Bilderzeugungsvorrichtungen den Holzkörper erfaßt haben.

Von Vorteil ist es, wenn das Bilddokument mit Datensätzen, vorzugsweise einem Erfassungsdatum und/oder einer Erfassungsuhrzeit und/oder Vermessungsdaten versehen wird.

Dadurch, daß das Bilddokument mit einem Erfassungsdatum und gegebenenfalls mit einer Erfassungsuhrzeit versehen bzw. verknüpft ist, läßt sich der Holzkörper auch zeitlich einer entsprechenden Fuhre oder einem entsprechenden Lieferanten zuordnen. Die Aufnahme der Vermessungsdaten in das Bilddokument bzw. eine entsprechende Verknüpfung des Bilddokumentes mit den Vermessungsdaten, z.B. der Länge, des Durchmessers und somit des Volumens, läßt weitere Rückschlüsse auf die Beschaffenheit des Holzkörpers bzw. dessen Wert oder Kaufpreis zu.

Eine Vorrichtung zur Durchführung des Verfahrens sieht in vorteilhafter Weise vor, daß um eine Fördereinrichtung zur Beförderung von Holzkörpern Bilderzeugungsvorrichtungen angeordnet sind, die zur Erstellung eines Bilddokumentes den Holzkörper optisch erfassen bzw. aufnehmen.

Eine derartige Vorrichtung zur Durchführung des Verfahrens hat sich als besonders vorteilhaft, einfach und kostengünstig herausgestellt.

Vorteilhafte Weiterbildungen und Ausgestaltungen der Erfindung ergeben sich aus den weiteren Unteransprüchen und aus dem nachfolgend anhand der Zeichnung prinzipmäßig dargestellten Ausführungsbeispiel.

Es zeigt:
- Fig. 1: eine schematische Seitenansicht auf das erfindungsgemäße Verfahren; und
- Fig. 2: eine schematische Darstellung des erfindungsgemäßen Erfassungs- und Abspeicherungsvorganges.

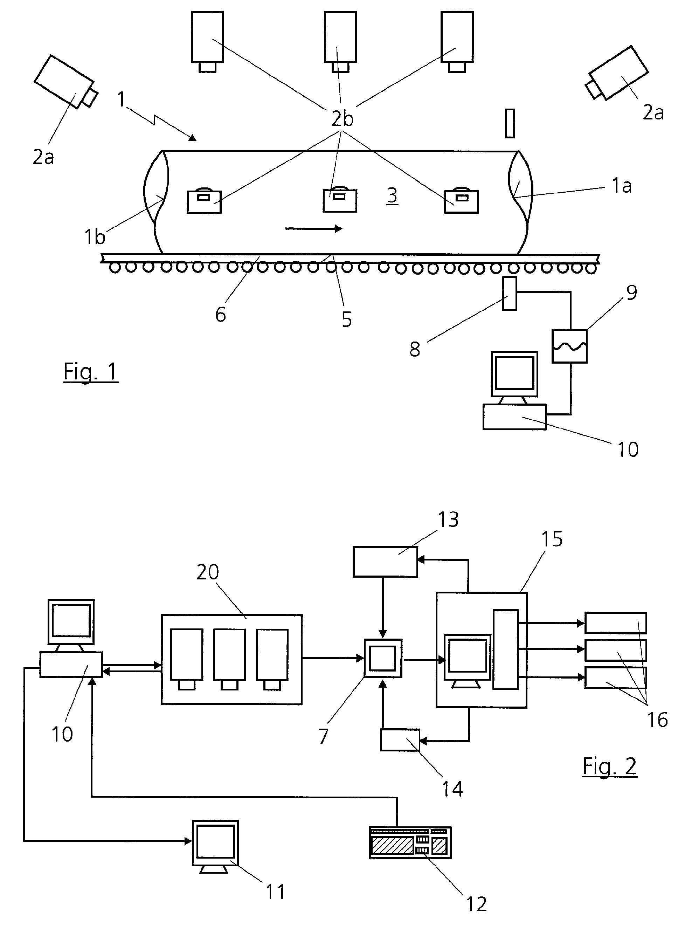

Wie aus Fig. 1 ersichtlich, weist das Verfahren zur Dokumentation, insbesondere zur optischen Qualitätsdokumentation, eines Holzkörpers 1 mehrere Bilderzeugungsvorrichtungen 2a, 2b auf, die den Holzkörper 1 erfassen bzw. aufnehmen. Der in dem Ausführungsbeispiel dargestellte Holzkörper ist dabei als Rundholzabschnitt 1 ausgebildet. Die Erfassung durch die Bilderzeugungsvorrichtungen 2a, 2b erfolgt dabei werkseingangsseitig in einem Sägewerk bzw. einer Sägeindustrie. Der Rundholzabschnitt 1 wird im allgemeinen direkt aus dem Wald von einem entsprechenden Lieferanten bzw. Forstunternehmen angeliefert. Die Erfassung des Rundholzabschnittes 1 kann sowohl im unbearbeiteten als auch im entrindeten Zustand erfolgen. Für die Dokumentation ist dabei lediglich entscheidend, daß sich die Qualität des angelieferten Rundholzabschnittes, insbesondere zur späteren Preisermittlung erkennen läßt.

In dem dargestellten Ausführungsbeispiel sind zwei stirnseitige Bilderzeugungsvorrichtungen 2a und jeweils drei längsseitige Bilderzeugungsvorrichtungen 2b an einer Längsseite 3 und in einem Winkel von 90° auf eine Oberseite 4 des Rundholzabschnittes 1 zur Erfassung desselben vorgesehen. Eine erste stirnseitige Bilderzeugungsvorrichtung 2a erfaßt dabei eine vordere Stirnseite 1a des Rundholzabschnittes 1 und eine zweite Bilderzeugungsvorrichtung 2a erfaßt eine hintere Stirnseite 1b des Rundholzkörpers 1.

Ein Einsatz von jeweils drei längsseitigen Bilderzeugungsvorrichtungen 2b an einer Längsseite 3 des Rundholzabschnittes 1 und in einem Winkel von 90° auf die Oberseite 4 hat sich als besonders geeignet herausgestellt. Bezüglich einer optimalen Qualitätsdokumentation ist eine derartige Anordnung vollkommen ausreichend, so daß ein Erfassen von weiteren Längsseiten 3 des Rundholzabschnittes 1 nicht notwendig ist.

Die in Fig. 1 dargestellten stirnseitigen Bilderzeugungsvorrichtungen 2a sind in vorteilhafter Weise in einem Abstand von 500 mm bis 8.000 mm, vorzugsweise 2.000 mm, zu einer Unterseite 5 des Rundholzabschnittes 1 mittig über einer Fördereinrichtung 6 für den Rundholzabschnitt 1 angeordnet. Die Fördereinrichtung 6 dient in bekannter Weise zur Beförderung der Rundholzabschnitte 1. Eine Anordnung der stirnseitigen Bilderzeugungsvorrichtungen 2a in dem beschriebenen Abstand hat sich unter Berücksichtigung der Qualität des Bildes, der Bildauflösung, des Erfassungswinkels und der Bildschärfe als besonders geeignet herausgestellt. Die Stirnseiten 1a, 1b des Rundholzabschnittes 1 lassen sich in besonders vorteilhafter Weise erfassen, wenn die stirnseitigen Bilderzeugungsvorrichtungen 2a in einem Blickwinkel von 35° bis 50° zu den Stirnseiten 1a, 1b des Rundholzabschnittes 1 angeordnet werden.

Vorteilhaft ist außerdem, wenn die Stirnseiten 1a, 1b jeweils wenigstens von einem nicht näher dargestellten Beleuchtungselement beleuchtet werden. Hierfür können beispielsweise Halogenscheinwerfer eingesetzt werden. Negative Lichteinflüsse bzw. optische Umwelteinflüsse lassen sich dadurch weitgehend ausschließen.

Darüber hinaus ist es von Vorteil, wenn der Sichtbereich der Bilderzeugungsvorrichtungen 2a, 2b in ebenfalls nicht dargestellter Weise, durch eine Abdeckung gegen Sonneneinstrahlung geschützt wird. Dadurch lassen sich die Rundholzabschnitte 1 in einfacher und kostengünstiger Weise mit relativ einfachen Bilderzeugungsvorrichtungen 2a, 2b in einer hohen Qualität erfassen. Dies kann beispielsweise durch eine Installation der Bilderzeugungsvorrichtungen 2a, 2b in einer geschlossenen lichtdichten Box erfolgen.

Als Bilderzeugungsvorrichtungen 2a, 2b können in einfacher Weise digitale Kameras verwendet werden. Dies hat sich auch bezüglich der Erstellung eines Bilddokumentes 7 als besonders vorteilhaft herausgestellt. Das Kamerasichtfeld für die Betrachtung der Stirnflächen 1a, 1b kann beispielsweise auf 600 mm x 600 mm ausgelegt werden, so daß auch Rundholzabschnitte 1 mit großen Stammdurchmessern erfaßbar sind. Um auch bei kleineren Stammdurchmessern eine maximale Abbildungsqualität zu erreichen, kann ein steuerbares Zoomobjektiv eingesetzt werden. Dies kann sowohl manuell in nicht näher dargestellter Weise durch eine Bedienperson als auch über einen Lichtschrankenvorhang angesteuert werden.

Die digitalen Kameras 2 können in einfacher und kostengünstiger Weise stationär um die Fördereinrichtung 6 aufgestellt bzw. angeordnet werden.

Wie aus Fig. 1 ersichtlich ist, weist das Verfahren zur Qualitätsdokumentation von Rundholzabschnitten 1 eine Lichtschranke 8 auf, die ein Ausgangssignal 9 generiert bzw. sendet, wenn der Rundholzabschnitt 1 die Lichtschranke 8 passiert. Das Ausgangssignal 9 kann dabei alle Kameras 2a, 2b bzw. zumindest die stirnseitigen Kameras 2a gleichzeitig auslösen. Die Lichtschranke 8 kann dabei auch als Lichtschrankenvorhang zur Ermittlung des Stammdurchmessers ausgebildet sein.

In einer alternativen Ausgestaltung kann dabei vorgesehen sein, daß das Ausgangssignal 9 einem entsprechenden Auswerterechner 10 zugeführt wird, der das Erfassen bzw. Aufnehmen des Rundholzabschnittes 1 steuert. Dabei kann auch vorgesehen sein, daß möglicherweise lediglich nur einige Kameras 2a, 2b gezielt ausgelöst werden.

Alternativ oder ergänzend dazu kann auch vorgesehen sein, daß das Speichern der Bilder durch einen Bediener erfolgt, der anhand der an einem Bildschirm angezeigten Bilder die Qualität der Rundholzabschnitte 1 beurteilt und per Tastendruck die Speicherung veranlaßt. Dies kann beispielsweise bei einer nicht pauschalen Erstellung der Bilddokumente 7 erfolgen, wenn der Bediener Hinweise auf mangelnde Qualität der Rundholzabschnitte 1 feststellt.

Wie aus Fig. 2 ersichtlich ist, kann eine Steuerung 20 der Kameras 2a, 2b durch den Auswerterechner 10 erfolgen. Die einzelnen von den Kameras 2a, 2b erzeugten Bilder können dabei auf einem Kontrollmonitor 11 angezeigt werden. Anhand dieses Kontrollmonitors 11 kann eine Bedienperson mittels einem entsprechenden Eingabeterminal 12, insbesondere bei einer nicht pauschalen Speicherung, eine entsprechende Auswahl durchführen bzw. in die Steuerung 20 bzw. in die Erstellung des Bilddokumentes 7 manuell eingreifen.

Das Bilddokument 7 wird digital, vorzugsweise im JPEG-Format, gespeichert und mit einem Identifizierungsschlüssel 13 versehen. Der Identifizierungsschlüssel 13 bzw. der Dateiname wird dabei, wie in Fig. 2 ersichtlich, durch die Fuhrenkennung, die verwendeten Bilderzeugungsvorrichtungen 2a, 2b und eine fortlaufende automatisch aufsteigende Numerierung gebildet. Somit ist eine eindeutige Zuordnung der einzelnen erfaßten Rundholzabschnitte 1 möglich. Zusätzlich zu dem Identifizierungsschlüssel 13 wird das Bilddokument 7 mit weiteren Datensätzen 14 versehen. Diese Datensätze 14 können beispielsweise das Erfassungsdatum, die Erfassungsuhrzeit und spezifische Vermessungsdaten des jeweiligen Rundholzabschnittes 1, wie beispielsweise die Länge, den Durchmesser und das Volumen, enthalten. Die zusätzlichen Datensätze 14 können entweder direkt auf dem Bilddokument 7 abgelegt bzw. mit diesem verknüpft werden.

Wie ebenfalls aus Fig. 2 ersichtlich, wird der Ablageort bzw. das Verzeichnis des Bilddokuments 7 von einem Leitrechner 15 bestimmt. Der Leitrechner 15 kann dabei die einzelnen Bilddokumente 7 über die aktuelle Fuhrenbezeichnung exakt einer entsprechenden Fuhrenstatistik 16 zuordnen. Die Fuhrenstatistik 16 kann einzelnen Lieferanten zugeordnet werden, so daß gezielt Dokumente zu den einzelnen Fuhren bzw. Lieferanten aufgefunden werden können. Zur Erzeugung des Identifizierungsschlüssels 13 kann ebenfalls der Leitrechner 15 verwendet werden, der dabei einen Textstream mit der aktuellen Fuhrenbezeichnung übermittelt. Dabei wird ein recheninterner Stammzähler zurückgesetzt, so daß mit jeder Fuhre bzw. Fuhrenkennung die fortlaufende Numerierung des Identifizierungsschlüssels 13 erneut bei beispielsweise 1 beginnt. Zur Kontrolle kann vorgesehen sein, daß der Stammzähler auf dem Kontrollmonitor 11 des Bedieners angezeigt wird, so daß dieser die Möglichkeit eines manuellen Eingreifens hat, um bei einer Störung den Zählerstand bzw. die fortlaufende Numerierung mit dem Leitrechner 15 zu synchronisieren.

In nicht dargestellter Weise können die Bilddokumente 7 über einen Netzwerkanschluß von einem externen Rechner abgegriffen werden und zentral auf Festplatten oder über einen Brenner auf CD gespeichert werden. Bei einer durchschnittlichen Größe jeder Bilddatei von ca. 100 kB (also bei zwei aufgenommenen Stirnseiten 1a, 1b 400 kB/Rundholzabschnitt 1) und einer CD-Speicherkapazität von 650 MB können pro CD die Bilder von 1.625 Rundholzabschnitten 1 gespeichert werden.

## Patentansprüche

1. Verfahren zur Dokumentation, insbesondere zur optischen Qualitätsdokumentation, eines Holzkörpers (1), insbesondere eines an ein Sägewerk gelieferten unbearbeiteten und/oder entrindeten Rundholzabschnittes, wobei der Holzkörper (1) mittels wenigstens einer Bilderzeugungsvorrichtung (2a,2b) erfaßt und als Bilddokument (7) abgespeichert wird.

2. Verfahren nach Anspruch 1,
**dadurch gekennzeichnet, daß**
eine vordere Stirnseite (1a) und/oder eine hintere Stirnseite des Holzkörpers (1) von jeweils wenigstens einer stirnseitigen Bilderzeugungsvorrichtung (2a) erfaßt wird.

3. Verfahren nach Anspruch 2,
**dadurch gekennzeichnet, daß**
die stirnseitigen Bilderzeugungsvorrichtungen (2a) in einem Abstand von 500 mm bis 8.000 mm, vorzugsweise 2.000 mm, zu einer Unterseite (5) des Holzkörpers (1) mittig über einer Fördereinrichtung (6) für den Holzkörper (1) angeordnet werden.

4. Verfahren nach Anspruch 2 oder 3,
**dadurch gekennzeichnet, daß**
die stirnseitigen Bilderzeugungsvorrichtungen (2a) in einem Blickwinkel von 20° bis 70°, vorzugsweise 35° bis 50°, zu den Stirnseiten (1a) des Holzkörpers (1) angeordnet werden.

5. Verfahren nach Anspruch 2, 3 oder 4,
**dadurch gekennzeichnet, daß**
der Holzkörper (1) an seinen Stirnseiten (1a) jeweils wenigstens von einem Beleuchtungselement beleuchtet wird.

6. Verfahren nach einem der Ansprüche 1 bis 5,
**dadurch gekennzeichnet, daß**
der Holzkörper (1) von jeweils wenigstens einem, vorzugsweise drei, längsseitigen Bilderzeugungsvorrichtungen (2b) wenigstens an einer Längsseite (3) und/oder in einem Winkel von 90° auf eine Oberseite (4) des Holzkörpers (1) erfaßt wird.

7. Verfahren nach einem der Ansprüche 1 bis 6,
**dadurch gekennzeichnet, daß**
beim Passieren des Holzkörpers (1) durch eine Lichtschranke (8) ein Ausgangssignal (9) zum Erfassen bzw. Aufnehmen des Holzkörpers (1) durch wenigstens einen Teil der Bilderzeugungsvorrichtungen (2a,2b) ausgelöst wird.

8. Verfahren nach einem der Ansprüche 1 bis 7,
**dadurch gekennzeichnet, daß**
das Bilddokument (7) digital, vorzugsweise im JPEG-Format, gespeichert und mit einem Identifizierungsschlüssel (13) versehen wird.

9. Verfahren nach Anspruch 8,
**dadurch gekennzeichnet, daß**
der Identifizierungsschlüssel (13) durch eine Fuhrenkennung und/oder die verwendeten Bilderzeugungsvorrichtungen (2a,2b) und/oder eine fortlaufende Numerierung gebildet wird.

10. Verfahren nach einem der Ansprüche 1 bis 9,
**dadurch gekennzeichnet, daß**
das Bilddokument (7) mit Datensätzen (14), vorzugsweise einem Erfassungsdatum und/oder einer Erfassungsuhrzeit und/oder Vermessungsdaten versehen wird.

11. Vorrichtung zur Durchführung des Verfahrens nach einem der Ansprüche 1 bis 10,
**dadurch gekennzeichnet, daß**
um eine Fördereinrichtung (6) zur Beförderung von Holzkörpern (1) Bilderzeugungsvorrichtungen (2a, 2b) angeordnet sind, die zur Erstellung eines Bilddokumentes (7) den Holzkörper (1) optisch erfassen bzw. aufnehmen.

12. Vorrichtung nach Anspruch 11,
**dadurch gekennzeichnet, daß**
das Bilddokument (7) mittels einem angeschlossenen Leitrechner (15) abspeicherbar ist.

13. Vorrichtung nach Anspruch 11 oder 12,
**dadurch gekennzeichnet, daß**
das Förderband mit einer Lichtschranke (8) versehen ist, die beim Passieren des Holzkörpers (1) ein Ausgangssignal (9) zur Erfassung des Holzkörpers (1) durch die Bilderzeugungsvorrichtungen (2a,2b) auslöst.
